Europäisches Patentamt

European Patent Office

Office européen des brevets

(11) Publication number: **0 501 445 A1**

## EUROPEAN PATENT APPLICATION

(21) Application number: **92103260.3**

(22) Date of filing: **26.02.92**

(51) Int. Cl.⁵: **C07K 15/08**, C07K 3/20, C12P 21/00, A61K 37/02

(30) Priority: **27.02.91 IL 97365**

(43) Date of publication of application:
**02.09.92 Bulletin 92/36**

(84) Designated Contracting States:
**AT BE CH DE DK ES FR GB GR IT LI LU MC NL PT SE**

(71) Applicant: **YEDA RESEARCH AND DEVELOPMENT CO. LTD.**
**P.O. Box 95**
**Rehovot 76100(IL)**

(72) Inventor: **Schwartz, Michal**
**Neve Metz 10, Weizmann Institute of Science**
**Rehovot(IL)**
Inventor: **Eitan, Shoshana**
**17 Helsinki Street**
**Tel Aviv(IL)**

(74) Representative: **Vossius & Partner**
**Siebertstrasse 4 P.O. Box 86 07 67**
**W-8000 München 86(DE)**

(54) **Pharmaceutical composition comprising interleukin-2.**

(57) Interleukin-2 or an Interleukin-2-like substance are effective for induction and facilitation of the regeneration of injured axons when administered to the site of the injury.

Rank Xerox (UK) Business Services

The present invention relates to the use of Interleukin-2 (IL-2) or IL-2-like substances for facilitating and enhancing regeneration of injured nerves of the central nervous system in mammals.

The central nervous system (CNS) of lower vertebrates has a high ability to regenerate after axonal injury, whereas mammalian central neurons have a low ability to regenerate. This poor regenerative capacity has been attributed, at least in part, to the presence of mature oligodendrocytes, which have been shown to be inhibitory to axonal growth.

In fish optic nerves, representative of a spontaneously regenerating CNS, regeneration was shown to be accompanied by the presence of regeneration-associated substances, which, when applied to injured adult rabbit optic nerves, facilitate their regeneration (Schwartz, M. et al., (1985) Science **228**, 601-603; Lavie, V. et al., (1990) J.Comp.Neurol. **298**, 293-315; European Patent No. 172987). An activity cytotoxic to oligodendrocytes was attributed to substances within these preparations, which presumably enable the fish optic nerve to overcome the inhibitory activity associated with oligodendrocytes (Sivron, T. et al., (1990) Glia **3**, 267-276). The cytotoxicity **in vitro** was shown to be not only to fish oligodendrocytes, but also to rat oligodendrocytes (Sivron, T. et al., see above and Cohen, A. et al.,(1990) Brain Res. **537**, 24-32). These substances are associated, directly or indirectly, with macrophages or other blood derived cells.

European Patent Application EP 415321 describes an oligodendrocyte cytotoxic factor present in the conditioned media of regenerating injured nerves of lower vertebrates, such as fish, but not in the conditioned media of intact nerves of lower vertebrates nor in the conditioned media of injured or intact nerves of mammals.

Interleukin-2 (IL-2) is a lymphokine known to be synthesized and secreted by T cells after activation with antigen or mitogen in the presence of IL-1 (Smith, K.A., (1988) Science **240**, 1169-1176). IL-2 in the immune system has been considered to be an important cytokine, responsible for either inhibition or progression of many immune responses (Liang, S.M. et al., (1989) Biochem.Biophys.Res.Commun. **165**, 1312-1318). In contrast, very little is known about the role of IL-2 in the brain. In the nervous system, some of the observations related to the effects of IL-2 on oligodendrocytes appear to be contradictory. Recent studies have attributed an inhibitory effect on oligodendrocytes to the cytokine IL-2 (Saneto, R.P. et al., (1986) Proc.Natl.Acad.Sci.USA **88**, 9221-9225; Saneto, R.P., et al., (1987) J.Neurosci.Res. **18**, 147-154), while other studies have shown a proliferative effect of IL-2 on oligodendrocytes (E.N. Benveniste and J.E. Merril (1986) Nature **321**, 610-613). IL-2 in mammals has been shown to be a product of lymphocytes (Smith, K.A. see above) and some reports have indicated that fish lymphocytes may have IL-2-like activity (Caspi, R.R. and Avtalion, R.R. (1984) Dev.Comp.Immunol. **8**, 51-60).

An association between IL-2 and injury in the CNS in general, and in the brain in particular, has also been pointed out (Nieto-Sampedro, M. et al., (1987) Neurochem.Res. 12, 723-727; Araujo, D.M., et al., (1989) Brain Res. **498**, 257-266). Nieto-Sampedro et al. found IL-2 activity after brain injury. Similarly Liang et al. (cited above) found IL-2 in brain lesions created by MPP[+](1-methyl-4-phenyl pyrimidine). In addition, up-regulation of IL-2 binding sites was observed by Araujo et al. in rat hippocampus as a result of injury. Nevertheless, no association between IL-2 and CNS regeneration has yet been suggested.

Other recent studies have also suggested that regeneration might be promoted by treatments that circumvent growth hindrance by oligodendrocytes, e.g., applications of a factor cytotoxic to oligodendrocytes such as TNF (European Patent Application EP 455093 and Schwartz, M. et al. (1991) Brain Res. **545**, 334-338) or of antibodies directed against the oligodendrocyte-associated inhibitors (Schnell, L. et al., (1990) Nature **343**, 269-272).

Robbins et al., J.Immunol., **138**, 8, 2593-7 (1987), have reported that stimulation of rat astrocytes **in vitro** resulted in the generation of a cytotoxic factor that is functionally similar to tumor necrosis factor. They also report that human recombinant tumor necrosis factor has cytotoxic activity directed against rat oligodendrocytes. Selmaj et al., Ann.Neurol., **24**, 4, 339-46 (1988) reported on the testing of recombinant human tumor necrosis factor (rhTNF) for its effect on myelinated cultures of mouse spinal cord tissue. They found that rhTNF induced delayed-onset oligodendrocyte necrosis and a type of myelin dilatation.

Despite substantial research efforts worldwide, no safe and effective means for causing CNS regeneration in mammals, and particularly humans, has yet been developed. Such a means, and particularly a pharmaceutical composition which can be injected at the site of desired regeneration would be greatly desirable in order to alleviate post-traumatic paraplegia or quadraplegia, blindness, deafness, surgically associated axotomy, etc.

It has now been found according to the present invention that Interleukin-2 and IL-2-like substances will facilitate axonal regeneration. It has been further found that the substance cytotoxic to oligodendrocytes originating from fish optic nerves, is an IL-2-like substance.

Accordingly, the present invention provides use of IL-2 and IL-2-like substances in the preparation of a medicament for induction and facilitation of the regeneration of CNS axons in mammals, particularly in humans.

It is an object of the present invention to provide a pharmaceutical composition comprising a unit dose of Interleukin-2 or an Interleukin-2-like substance and a pharmaceutically acceptable carrier or excipient in a quantity effective for induction and facilitation of the regeneration of injured axons when administered to the site of the injury.

It is a further object of the present invention to provide a method for inducing and facilitating the regeneration of injured central nervous system axons in a mammal comprising administering to the site of the injury an effective quantity of Interleukin-2 or an Interleukin-2-like substance.

It is still a further object of the invention to provide oligodendrocyte cytotoxic factor derived from fish optic nerves, an Interleukin-2-like substance, in substantially purified form.

Figure 1 shows that anti-IL-2 antibodies neutralize the cytotoxic effect of fish soluble substances in a conditioned medium (CM) on oligodendrocytes.

Figure 2 depicts Western blot analysis of the fish optic nerve soluble substances using mouse anti-human IL-2 monoclonal antibodies.

Figure 3 shows affinity purification of the IL-2-like factor from fish optic nerve conditioned medium.

Figure 4 shows reduction in oligodendrocyte number by recombinant mouse IL-2 in rat brain oligodendrocyte cultures.

Figure 5 shows reduction in the number of mature oligodendrocytes (GalC-labeled) caused by recombinant mouse IL-2.

Figure 6 shows newly growing fibers in transected IL-2-treated nerves.

Regeneration of axons at the site of a CNS injury in a mammal is induced and facilitated by the administration to the area of the injury of IL-2 or an IL-2-like substance. While it is preferred that the IL-2 or IL-2-like substance be of the same species as the mammal being treated, this is not critical.

An IL-2-like substance according to the present invention is a substance which is crossreactive with antibodies against IL-2. This definition includes muteins and modified IL-2 molecules having amino acid residues added, deleted or substituted as compared to native human IL-2, as well as IL-2 derivatives which have added moieties or other peptide sequences to improve their physical properties for use in a pharmaceutical composition, as long as such muteins and modified proteins have the property of inducing and facilitating the regeneration of injured mammalian axons **in vivo**. This function may be tested, without undue experimentation, by means of an **in vitro** test for selective cytotoxicity to oligodendrocytes.

The IL-2 used according to the present invention includes native IL-2 of any species and recombinant IL-2 of any species. The IL-2 may be obtained by any convenient technique, such as by the process of R.J. Robb et at. (1983) Proc.Natl.Acad.Sci.USA **80**, 5990. It is preferably obtained by recombinant DNA technologies, for example as described by T. Taniguchi et al., (1983) Nature **302**, 305-310 or Devos, R. (1983) Nucleic Acids Research **11**, 4307-4323. Recombinant human IL-2 (rhIL-2) is presently commercially available. Some IL-2 muteins may be obtained as described in US Patent No. 4,518,584.

A preferred IL-2-like substance used according to the invention is the oligodendrocyte cytotoxic factor derived from fish optic nerves in substantially purified form. The factor, originally disclosed in EP 415321 is here, for the first time, fully characterized and obtained in purified form.

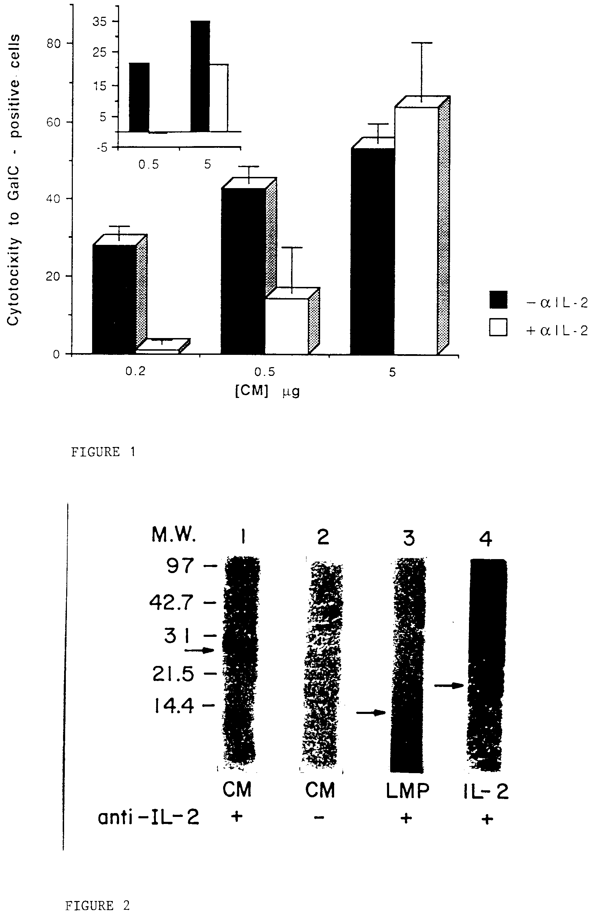

The oligodendrocyte cytotoxic factor originating from the fish optic nerves was found, according to the present invention, to be an IL-2-like molecule, as confirmed by the following: (i) it was purified by affinity chromatography of anti-IL-2 antibodies ; (ii) antibodies against IL-2 neutralized the cytotoxic activity of the conditioned medium derived from regenerating fish optic nerves; (iii) Western blot analysis revealed the presence of an IL-2 immunoreactive band of 28 kDa in the fish conditioned medium; and (iv) recombinant mouse IL-2 had a selective cytotoxic effect **in vitro** on oligodendrocytes but not on astrocytes. The apparently higher potency of the IL-2-like substances in the fish conditioned medium than that of the recombinant mouse Il-2 strongly suggests that the specificity of the activity is determined by the tissue and not by the species, i.e., the CNS-derived fish IL-2 is probably different from the recombinant IL-2, which is immune-derived, indicating that the active molecule from fish optic nerves is a modification of the immune-derived IL-2. This is further substantiated by the results, shown in Figure 2, that the IL-2 immunoreactive substance in fish blood lymphocytes is a polypeptide of about 14 kDa, not a 28 kDa polypeptide as the IL-2-like substance in the nerve.

The oligodendrocyte cytotoxic factor derived from fish optic nerves in substantially purified form is an IL-2-like substance with a molecular weight of about 28 kDa as determined by Western blot analysis. It is present in the conditioned media of regenerating injured nerves of lower vertebrates, such as fish, but not in the conditioned media of intact nerves of lower vertebrates or of injured or intact nerves of mammals. It is selectively toxic to the oligodendrocyte lineage but not to type-1 astrocytes and fibroblast cells. This cytotoxic activity is neutralized by antibodies directed against IL-2.

3

The purified fish oligodendrocyte cytotoxic factor is obtained by affinity purification. Conditioned medium containing the factor prepared by incubating serum-free medium with crushed regenerating fish optic nerves is applied to a column containing antibodies directed against IL-2, the bound substances are eluted with a suitable solvent, thus obtaining the fractions containing the factor of 28 kDa as tested by their cytotoxicity to oligodendrocytes and by Western blot anlysis.

Any anti-IL-2 antibodies can be used in the affinity purification process. Preferably, anti-human IL-2 antibodies, and particularly mouse monoclonal antibodies against recombinant human IL-2 are used, coupled to a suitable resin, such as polyacrylhydrazide agarose. Elution of the bound substances is carried out, for example, with glycine.

The IL-2 or IL-2-like substance is used in the present invention in a quantity and purity sufficient to facilitate regeneration of CNS axons in mammals, particularly humans. Since the purified oligodendrocyte cytotoxic factor was shown to be more potent than IL-2, it will be used in lower amounts than IL-2. They are administered in any manner which is suitable to bring it to the vicinity of the injured axons to be regenerated. Preferably, they are injected in a pharmaceutically acceptable liquid carrier directly to the site.

Alternatively, an implant bearing the IL-2 or IL-2-like substance may be surgically inserted. Such an implant may consist of any material, such as nitrocellulose, which will absorb the active substance like a sponge and slowly release it at site of implantation. Other means of delivery will be apparent to those skilled in this art and are intended to be comprehended within the scope of the present invention.

The amount of the IL-2 or IL-2-like substance to be administered to any given patient depends on a variety of factors, such as the injury being treated, the site of injured axons it is wished to regenerate and the condition of the patient. Typically, however, the IL-2 or IL-2-like substance is administered as a single injection or soaked onto nitrocellulose or any other adsorbable carrier. Precise dosages will be empirically determined.

The IL-2 or IL-2-like substance is preferably administered as soon as possible after the injury being treated. Thus, it is preferably used for acute injury rather than chronic injury. It will be more difficult to facilitate regeneration in accordance with the present invention the longer a period of degeneration has existed.

While the administration of IL-2 or IL-2--like substance alone shows good results, such treatment may be combined with any type of concomitant therapy which may tend to augment its effects. For example, irradiation of the injury site with low energy laser, preferably He-Ne laser (5 min/day, 35 mW) can delay the post-traumatic process of the degeneration and thereby delay scar formation. See Assia et al., Brain Res., **476**, 205-212 (1988).

The various injuries which can be treated in accordance with the present invention are myriad and will be readily apparent to those of ordinary skill in the art. Without limitation, there may be mentioned neural trauma, diseases involving acute or subacute damage to CNS axon caused by pressure or ischemia, e.g. glaucoma, anterior ischemic optic neuropathy, spinal cord injuries, injuries to the optical nerve or to the aural nerves, etc. Injury to CNS neurons during neurosurgery or caused by tumors may also be treated by means of the present invention.

For the purpose of the present invention, the IL-2 or IL-2-like substance may be formulated with any pharmaceutically or veterinarily acceptable carrier or diluent. They may be presented as an aqueous solution, for example as a sterile aqueous solution. A solution or powder containing IL-2 or IL-2-like substance may be stabilized by means of a stabilizing agent. It may be formulated in a unit dosage injectable form (solution, suspension, emulsion) preferably in a pharmaceutically acceptable carrier medium that is inherently non-toxic and non-therapeutic. Examples of such vehicles include saline, Ringer's solution, dextrose solution, mannitol and normal serum albumin. Non-aqueous vehicles such as fixed oils and ethyl oleate may also be used. The carrier medium may contain minor amounts of additives such as substances that enhance isotonicity, solubility and/or chemical stability, e.g., buffers, detergents and preservatives. Various formulations for IL-2 are already known for other indications. Such formulations may also be used for the purpose of the present invention as long as the desired function of the IL-2 is not affected thereby.

According to the invention, there is provided immunochemical evidence that an IL-2-like substance is present among the soluble substances derived from the fish optic nerves, and its level increases after injury. This substance was characterized and identified as the previously described oligodendrocyte cytotoxic factor. Antibodies directed against mammalian IL-2 neutralize its cytotoxic effect on rat brain oligodendrocytes. Moreover, recombinant mouse IL-2 is shown to be cytotoxic to mature rat oligodendrocytes **in vitro** and to facilitate axonal growth of injured rabbit optic nerves **in vivo**.

The following Examples will illustrate the invention.

**Examples**

**Experimental Procedures**

    **a. Preparation of soluble substances derived from regenerating fish optic nerves.** Carp (**Cyprinus carpio,** 800-1200 g, Tnuva, Israel) allowed to acclimate for 1 day were anesthetized with 0.05% tricaine methanesulfonate (Sigma, St. Louis, MO, USA) and their optic nerves crushed with forceps (for 30 secs). Care was taken to injure only the nerves, leaving the surrounding tissue intact. On the 8th day after injury, the crushed-regenerating nerves were dissected out and incubated in serum free medium (DMEM, Gibco) for 1.5 h (4 nerve segments/300 $\mu$l of medium) at 25°C. The resulting medium, defined as conditioned medium (CM), was then collected and its protein content determined by the Bradford's assay. Aliquots of 50 $\mu$l or 500 $\mu$l were stored at -70°C.

    **b. Preparation of medium conditioned by fish lymphocytes.** Fish were anesthetized with 3-aminobenzoic acid ethyl ester (Sigma). After the carotid artery in the eye cavity was cut, blood was collected from the socket into a tube containing heparin sulphate (100 U/ml; BDH Chemicals). The blood was diluted with an equal volume of phosphate buffered saline (PBS) and allowed to stand for 5 min before being layered on top of the Ficoll or Percoll solutions. Immediately before use, a working solution of 29.4% sodium diatrizoate (Sigma) stock was mixed with 70.6% Ficoll stock, to make a density of 1.06 g/ml. When a Percoll solution was used, a stock solution of Percoll (1.06 g/ml) was made by mixing 44.6 ml Percoll (Pharmacia), 10 ml 100 mM citric acid (Merck), 10 ml 5% bovine serum albumin (BSA; Sigma), and 35.4 ml PBS. Fifteen milliliters of the solution was placed into a 50 ml sterilized Corex (Corning) centrifuge tube. Thirty milliliters of diluted blood were carefully layered on top of the solution with a small pipet. The tubes were capped and centrifuged in swinging buckets at 800 X g for 30 min at 20°C.

    After centrifugation, white blood cells were found in the interphase, where they could easily be removed using a Pasteur pipet. The white blood cells were collected into a 50 ml tube and washed twice with PBS. The cells were suspended in a small volume of L-15 medium containing penicillin and streptomycin (100 U/ml), and then counted. The cells were diluted to $20 \times 10^7$ cells/ml and 10 ml aliquots were placed into 75 cm$^2$ flasks (Falcon). The flasks were left for 1 h at 20°C and the nonadherent cells were removed and placed into a second flask. This was repeated, and after 1 h the nonadherent cells were placed into a third flask. The first and second flasks were washed twice with 100 ml of PBS with vigorous shaking. The first flask contained a macrophage enriched culture and the third flask contained a lymphocyte enriched culture. Ten milliliters of medium was added and all flasks were incubated for 8 h, after which time all the supernatants were collected and centrifuged at 2000 X g for 5-15 min. The supernatants were then concentrated 25-100- fold in a Centricon or Amicon unit (Amicon Corp.). The samples were then stored at 4°C.

    **c. Preparation of rat brain oligodendrocyte cultures and immunofluorescence staining.** For the preparation of enriched oligodendrocyte cultures, neonatal rat brains (2 days old) were excised [2 brains in 2 ml of Leibowitz medium (L-15); Gibco] and chemically dissociated by $3 \times 10^4$ U/ml trypsin (Sigma) in DMEM ($Ca^{2+}$ and $Mg^{2+}$ free) containing 1 mM ethylenediaminetetraacetic acid (EDTA). Mechanical dissociation was carried out prior to 10 min incubation at 37°C with the trypsin solution. The cells were then transferred into 15 ml conical tubes containing 1 ml of solution of 74 U/ml DNase (Sigma), 5200 U/ml soybean trypsin (Sigma) and 3 mg BSA, incubated for 1 min at room temperature, added to 10 ml of medium, and subsequently washed 3 times in DMEM. After the last wash, the cells were suspended in 10 ml DMEM containing 5-10% fetal bovine serum (FBS; Sigma-heat inactivated at 56°C for 30 min), passed through mesh and seeded in 85 mm$^2$ flasks (Nunc), previously coated overnight at 37°C with 20 $\mu$g/ml poly-L-lysine (PLL, MW 100000; Sigma). The medium was changed first twenty-four hours after cell seeding and then once every 2-3 days thereafter. On the 8th day after seeding, the cells were incubated with shaking for 4-6 h, the supernatant was removed and the remaining cells further incubated in 10 ml medium (DMEM plus 5-10% FBS) for several hours followed by an overnight shaking. The cells that were removed by the shaking were collected and centrifuged, and the pelleted cells were then resuspended in 1-2 ml of serum-free medium (Bottenstein, J.E. et al., (1979) Proc.Natl.Acad.Sci.USA 76, 514-517).

    The thus obtained enriched oligodendrocyte cultures recovered cells were seeded on glass cover-slips (13 mm; $3 \times 10^5$ cells/well) previously coated with PLL (20 $\mu$g/ml), The glass coverslips were placed in a 24-well plate (Nunc). During seeding, 50 $\mu$l of cell suspension was initially applied to each coverslip and left for 30 min at 37°C, to allow attachment. Subsequently, the cells were washed with DMEM and then 500 $\mu$l of defined medium (Raff's modification to Bottenstein's and Sato's defined medium) was added. After 48 h, the seeded cells were treated with the conditioned medium or with conditioned medium preincubated for 2 h either with rabbit anti-human IL-2 antibodies (Genzyme, Inc.) or with control antibodies (rabbit antineurofilaments). The number of mature oligodendrocytes, i.e., galactocerebroside (GalC) positive cells, was determined by immunofluorescence as follows: Cells were thoroughly washed with Hank's balanced salt solution (HBSS) containing 2% FBS, heat inactivated and incubated for 30 min at 37°C with 50 $\mu$l of the monoclonal anti-GalC antibodies (IgG3, hybridoma supernatant diluted 1:5 in DMEM, Serotec). At the end of the incubation, the cells were washed and further incubated with 50 $\mu$l of fluoresceinconjugated goat anti-mouse IgG3 (1:50 in DMEM), and then washed and fixed in methanol for 10 min at -20°C. At a final washing the cells were coated with glycerol containing 22 mM 1,4-diazobicyclo(2,2,2)octaine. As controls, coverslips were used which underwent the same staining procedure, except for the primary antibodies which were omitted. Coverslips were placed on the glass slides, sealed with nail polish and stored at 4°C. Cells were counted over the entire coverslip.

**d. Western blot analysis of IL-2 immunoreactive proteins.** Samples were electrophoresed on SDS-PAGE. The gel was blotted onto nitrocellulose for 2 h at 200 mA. The nitrocellulose was incubated for 2 h at 37°C with PBS containing 5% milk, and then washed in PBS.

The blot was incubated with rabbit anti-IL-2 antibodies for 2 h at 37°C, then washed three times, 5 min each time, in PBS containing 0.05% Tween-20. Finally,the blot was incubated for 2 h at 37°C with [$^{125}$I]-labeled goat antirabbit antibodies ($10^6$ cpm/ml), washed three times with PBS containing 0.05% Tween-20, dried and autoradiographed.

**Example 1. Cytotoxicity of medium conditioned by regenerating fish optic nerves on oligodendrocytes can be neutralized by anti-IL-2 antibodies.**

Medium conditioned by regenerating fish optic nerves was treated with antibodies against IL-2 to examine whether this would result in a loss of cytotoxic activity. It was found that antibodies directed against IL-2 neutralized the cytotoxic effect of the conditioned medium.

The neutralization of the cytotoxic effect of fish soluble substances on oligodendrocytes by rabbit polyclonal antibodies directed against recombinant human IL-2 antibodies was assessed. The results are shown in Figure 1. After 48 h conditioned medium, either alone or preincubated with rabbit polyclonal antibodies against recombinant human IL-2, was added to the oligodendrocyte cultures and incubated for a further 48 h. Antibodies directed against neurofilaments were used as control. Oligodendrocytes were identified by immunofluorescence. The highest concentration of the soluble substances derived from regenerating fish optic nerves (5 $\mu$g protein) resulted in about 60% cytotoxicity and no neutralization with anti-IL-2 antibodies; 0.5 $\mu$g of conditioned medium caused 42% cytotoxicity, half of which could be neutralized by the antibodies; complete neutralization with the same amount of antibodies could be obtained when only 0.2 $\mu$g of the conditioned medium were applied. Results are expressed as percent cytotoxicity, in relation to cytotoxicity-free control cultures treated with the antibodies only (100% survival, no cytotoxicity). All experiments were repeated 3 times and were carried out using 0.1 $\mu$g of anti-IL-2 antibodies (IgG). The results of one experiment are given in this figure; CM designates conditioned medium. The absolute total number of GalC positive cells counted in each coverslip ranged from 300 to 500 in the various experiments. The inset shows basically the same experiments carried out with 10-fold more anti-IL-2 antibodies, i.e., 1 $\mu$g IgG.

As can be seen in the figure, antibodies against recombinant mouse IL-2 neutralized the cytotoxic activity of the conditioned medium. When the amount of antibodies was kept constant (0.1 $\mu$g IgG fraction; 1 $\mu$g neutralizes 1 U of IL-2) the neutralization achieved was a function of the concentration of conditioned medium applied, with complete neutralization at the lowest concentration tested, i.e., 0.2 $\mu$g/ml total protein. A 10-fold higher amount of antibodies (1 $\mu$g IgG fraction) resulted in neutralization of higher concentrations of conditioned medium (i.e., 0.5 and 5 $\mu$g/ml) (Fig. 1, inset). According to these results, and based on the potency of the IL-2 antibodies used for neutralization in the present assay, 1 $\mu$g of the conditioned medium is estimated to contain 0.5-2 U of biologically active IL-2-like molecules.

**Example 2. Characterization of the IL-2-like molecule from fish conditioned medium**

In order to determine the size of the IL-2-like molecule, the fish optic nerve conditioned medium containing soluble substances was subjected to Western blot analysis using mouse monoclonal antibodies directed against human IL-2. Optic nerve conditioned media (CM, 400 $\mu$g), media conditioned by fish blood lymphocytes (LMP, 16 $\mu$g), and mouse IL-2 (75 ng) were electrophoresed on SDS-PAGE. Lane 1 contains conditioned medium (CM) incubated with anti-IL-2 antibodies. Lane 2 is the control slot containing conditioned medium (CM) reacted with second antibodies only. Lane 3 is a slot containing medium conditioned by fish lymphocytes (LMP) incubated with the anti-IL-2 antibodies. The arrows at the left of each lane (excluding lane 2) point to the IL-2 immunoreactive bands in each slot. Molecular weight markers were electrophoresed on the same gel and are marked on the gel.

Figure 2 shows the presence of a single IL-2 immunoreactive band at a molecular weight of 28 kDa. Since lymphocytes are known to produce IL-2, the interaction of the same antibodies was compared with fish lymphocytes, under the same experimental conditions. As shown in Figure 2, a single IL-2 immunoreactive band of approximately 14 kDa was observed. These results suggest that IL-2, or a molecule with which IL-2 antibodies cross-react, might be responsible for the cytotoxic effect on oligodendrocytes.

**Example 3. Affinity Purification of the oligodendrocyte cytotoxic factor from fish conditioned medium**

Purification of the cytotoxic substances was carried out by the use of an IL-2 affinity column as follows.

Mouse monoclonal antibodies against recombinant human IL-2 antibodies were coupled to polyacrylhydrazide agarose (BioMakor, Israel). Using the procedure of Wilchek and Miron (Meth.Enzymol. (1974) **34**, 72-76), 0.1 ml of packed resin was coupled to 0.5 mg of antibodies. Purification was carried out as follows: Conditioned medium (50 $\mu$l) was added to the antibody-coupled packed resin, to which anti-IL-2 antibodies were coupled and incubated for 2 h at 37°C. The supernatant was then collected and the remaining resin washed 3 times, each time with 1 ml of PBS. Elution was carried out with 50 $\mu$l of glycine (0.2 M, pH 2.7) with shaking for 10 min, at room temperature. The eluted material (ELU) was collected into 10 $\mu$l Tris buffer (1 M, pH 8.0). The fractions containing the IL-2-bound substances were tested for their cytotoxic effects on oligodendrocytes by the use of the colorimetric MTT (3-(4,5 dimethylthiazol-2-yl)-2,5-diphenyl tetrazolium bromide) assay (Sigma) for assessment of the number of oligodendrocytes and hence of the cytotoxicity (Mosmann, T. (1983) J.Immunol.Meth. **65**, 55-63).

The assay was carried out as follows: Cells were treated with the conditioned medium alone or with the bound substances eluted from the column of anti-IL-2 antibodies (ELU). The final dilutions of the eluted substances (ELU) used in the assay corresponded to those of the crude conditioned medium. Following incubation for 48 h, 10 $\mu$l of MTT was added for 3 h; the medium was then removed and 100 $\mu$l of 0.04 N HCl in isopropanol was added. The cells were gently shaken until all crystals had dissolved, and their absorbance was recorded at 550 nm against 600 nm as a reference wavelength.

Cytotoxic activity on oligodendrocytes, assessed by the colorimetric MTT assay, was recovered from the medium conditioned by regenerating fish optic nerves (Experimental Procedure (a)) by elution of the substances bound to the anti-IL-2 antibodies, which had been coupled to the column. Controls were untreated cultures or cultures treated with medium containing the elution buffer (Buffer), thus ruling out the possibility that any cytotoxicity in the eluate might have resulted from the buffer used for elution of the bound material from the column. The experiment, which was repeated 3 times, was performed in triplicate and the results are presented as means ± SD of the untreated culture values, representing 100% survival. Analyses by the repeated measures method revealed that the effects of the eluted substances (ELU) differed significantly (P<0.05) from those of the two corresponding control cultures.

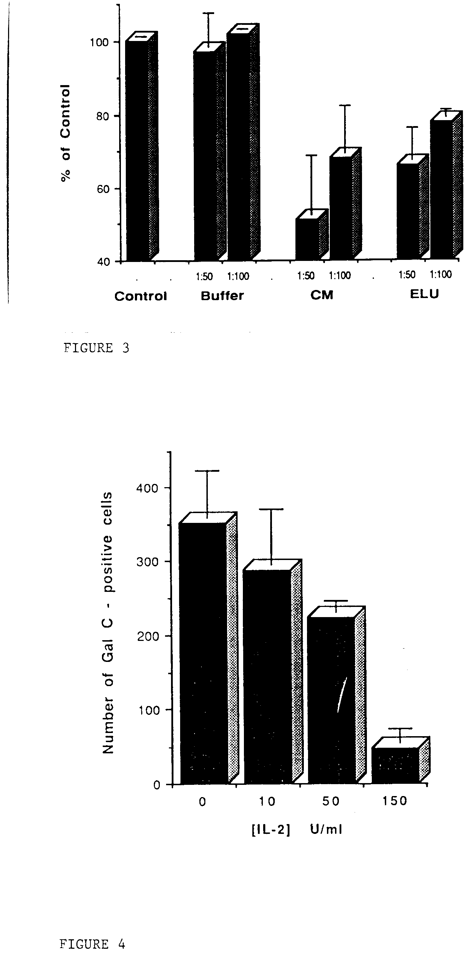

As shown in Figure 3, the IL-2-bound substances eluted from the column were cytotoxic to oligodendrocytes. The small scale of the purification did not permit calculation of the specific activity of the eluate. However, in view of the known limits of the protein detection we could estimate that the specific activity of the eluate was at least $10^3$-fold higher than that of the conditioned medium. The eluate, which was found to retain the cytotoxic activity, was also subjected to Western blot analysis using anti-IL-2 antibodies, which revealed the presence of the original 28-kDa immunoreactive polypeptide (data not shown). These results thus link the IL-2 immunoreactive 28-kDa protein and the cytotoxic activity.

**Example 4. Recombinant mouse IL-2 is selectively toxic to oligodendrocytes.**

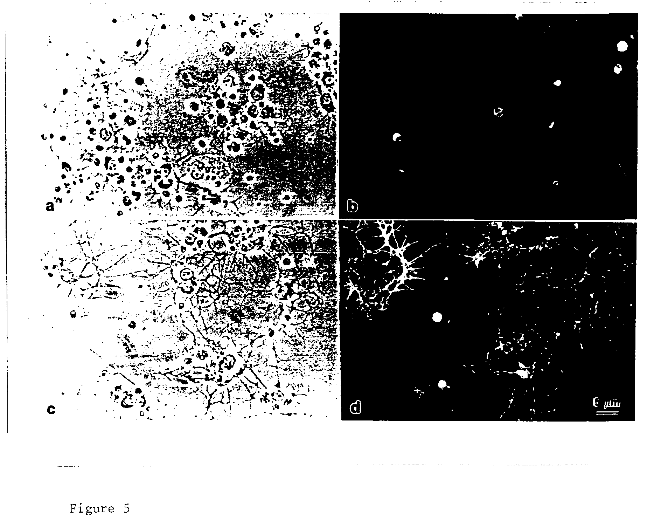

Recombinant mouse IL-2 (0-150 U/ml) was applied to enriched cultures of rat oligodendrocytes (prepared as described in Experimental Procedures) 48 h after seeding of the cells. The number of cells was assessed by the use of antibodies against GalC. The subsequent effect on the number of mature oligodendrocytes was determined by immunofluorescent assay, wherein the number of labeled cells was counted using a fluorescence microscope. Untreated cells were used as control (no cytotoxicity). As can be seen in Fig. 1,IL-2 caused a significant reduction in the number of mature oligodendrocytes, as was reflected by the number of galactocerebroside (GalC) positive cells. At high IL-2 concentrations the number of mature oligodendrocytes, as reflected by the number of GalC positive cells, was significantly reduced (88.6 ± 15.3% cytotoxicity at 150 U/ml; P<0.0005 by ANOVA) (Fig. 4). Representative micrographs showing the effects of recombinant mouse IL-2 (200 U/ml) on the number of mature oligodendrocytes (GalC-labeled) are shown in Figure 5. (b, d) Fluorescent micrographs of IL-2 treated (b) and untreated (d) cells, respectively, stained with anti-GalC antibodies. Respective phase micrographs of the fields shown in (b) and (d) are seen in (a) and (c). The cells which were spared were processless (Fig. 5b) rather than process-bearing cells (Fig. 5d). However, the amount of recombinant mouse IL-2 required for demonstrable cytotoxic activity on oligodendrocytes was far higher than the estimated amount in the conditioned medium. It is therefore possible that the IL-2 derived from fish is a modification of the immune-derived IL-2 and thus has a higher affinity for rat oligodendrocytes than that of the recombinant mouse IL-2 for rat oligodendrocytes. To rule out the possibility that the observed effect of the IL-2 on oligodendrocytes is due to nonspecific cytotoxicity, IL-2 was applied also to cultures of astrocytes, i.e., rat brain monolayers of flat cells. As expected, no cytotoxicity was observed (Table 1).

TABLE 1

| IL-2 U/ml | 5000 Cells/Well | | 10,000 Cells/Well | | 30,000 Cells/Well | |
|---|---|---|---|---|---|---|
| | CPM ±S D | % ±S D | CPM ±S D | % ±S D | CPM ±S D | % ±S D |
| 0 | 28030 ±518 | 100±2 | 48776 ±5954 | 101±15 | 55727 ±5137 | 100±11 |
| 0.1 | 33632 ±2351 | 120±7 | 53040 ±3639 | 109±13 | 60345 ±793 | 108±8 |
| 1 | 31084 ±984 | 110±3 | 55740 ±1436 | 115±12 | 58231 ±86 | 105±8 |
| 10 | 33384 ±5181 | 119±16 | 51745 ±5317 | 107±14 | 62328 ±103 | 112±9 |
| 50 | 31879 ±2957 | 113±9 | 53548 ±3251 | 110±13 | 56601 ±658 | 102±8 |
| 200 | 30248 ±1052 | 107±3 | 50064 ±2219 | 103±11 | 57380 ±2771 | 103±9 |

**Example 5. IL-2 activity in vivo**

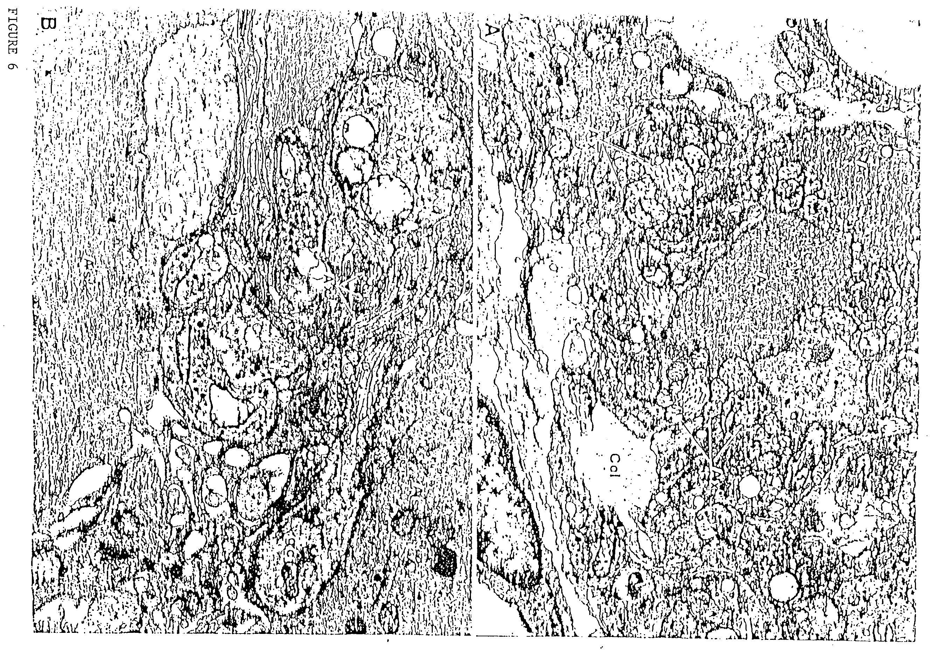

In order to test whether IL-2 can substitute the conditioned medium for the **in vivo** effect on axonal growth facilitation, recombinant mouse IL-2 was applied to injured adult rabbit optic nerves. Application was carried out using two experimental paradigms. One experimental paradigm involved transected adult rabbit optic nerves and application of IL-2 soaked into nitrocellulose (Lavie, V. et al., (1990) J.Comp.Neurol, **298**, 293-315). The second, involved a severe crush of the optic nerves and injection of IL-2, in a soluble form, into several sites along the nerves, from the site of the injury, distally. Assessment of the subsequent growth involved analysis by transmission electron microscopy and by anterograde transport of horseradish peroxidase (HRP) injected to the optic disc (Lavie et al., see above). The observed growth is depicted in Fig. 6. As can be seen, in the IL-2 treated, injured rabbit optic nerves 2 in distal to the site of the injury, abundant nonmyelinated axons, and growth cones, were observed. These were previously described (Lavie et al., see above) as characteristic of newly growing axons. No viable axons were observed in the control injured but untreated animals.

**Claims**

1. Use of Interleukin-2 or of an Interleukin-2-like substance for the preparation of a medicament for induction and facilitation of the regeneration of injured axons of the central nervous system.

2. Use according to claim 1 of human Interleukin-2.

3. Use according to claims 1 or 2 of recombinant human Interleukin-2.

4. Use according to claim 1 of a mutein of Interleukin-2.

5. Use according to claim 1 of oligodendrocyte cytotoxic factor derived from fish optic nerves in substantially purified form.

6. Oligodendrocyte cytotoxic factor derived from fish optic nerves in substantially purified form having the following characteristics:

i it is water-soluble;

ii it is present in the conditioned media of regenerating injured nerves of lower vertebrates, such as fish, but is not present neither in the conditioned media of intact nerves of lower vertebrates nor in the conditioned media of injured or intact nerves of mammals;

iii it is an Interleukin-2-like substance;

iv it is selectively toxic to the oligodendrocyte lineage, but not to other cells, such as type-1 astrocytes and fibroblast cells;

v its cytotoxic activity to oligodendrocytes is neutralized by antibodies directed against Interleukin-2;

vi it is purified from the conditioned medium of regenerating fish optic nerves by affinity chromatography with antibodies directed against Interleukin-2; and

vii it has a molecular weight of about 28 kDa as determined by Western blot analysis.

7. A process for the production of substantially purified oligodendrocyte cytotoxic factor derived from fish optic nerves, which process comprises:

a. subjecting conditioned medium of regenerating fish optic nerves to affinity chromatography with anti-IL-2 antibodies;

b. eluting the bound substances with a suitable solvent, and

c. recovering the purified factor having a molecular weight of about 28 kDa in the eluted fractions presenting selective cytotoxic activity to oligodendrocytes.

8. A process according to claim 7 wherein in step (a) said anti-IL-2 antibodies are anti-human IL-2 antibodies.

9. A process according to claim 7 or 8 wherein in step (a) said anti-IL-2 antibodies are mouse monoclonal antibodies against recombinant human IL-2.

10. A process according to any of claims 7 to 9 wherein in step (b) said suitable solvent is glycine.

FIGURE 1

FIGURE 2

FIGURE 3

FIGURE 4

Figure 5

FIGURE 6

## DOCUMENTS CONSIDERED TO BE RELEVANT

EP 92103260.3

| Category | Citation of document with indication, where appropriate, of relevant passages | Relevant to claim | CLASSIFICATION OF THE APPLICATION (Int. Cl.5) |
|---|---|---|---|
| A | CHEMICAL ABSTRACTS, vol. 112, no. 11, March 1990, Columbus, Ohio, USA GIULIAN, DANA et al. "The role of mononuclear phago-cytes in wound healing after traumatic injury to adult mammalian brain." page 582, column 1, abstract-no. 96 644t & J. Neurosci. 1989, 9(12), 4416-29 -- | 1-10 | C 07 K 15/08 C 07 K 3/20 C 12 P 21/00 A 61 K 37/02 |
| D,A | CHEMICAL ABSTRACTS, vol. 107, no. 11, September 14, 1987, Columbus, Ohio, USA NIETO-SAMPENDRO, MANUEL et al. "Interleukin-2-like activity in injured rat brain." page 552, column 1, abstract-no. 95 041d & Neurochem.Res. 1987, 12(8), 723-7 -- | 1-4 | |
| A | CHEMICAL ABSTRACTS, vol. 109, no. 13, September 26, 1988, Columbus, Ohio, USA GIULIAN, DANA et al. "Inter-leukin-1 injected into mammalian brain stimulates astrogliosis and neovascularization." page 471, column 1, abstract-no. 108 727a & J. Neurosci. 1988, 8(7), 2485-90 -- | 1-10 | TECHNICAL FIELDS SEARCHED (Int. Cl.5) C 07 K C 12 P 21/00 A 61 K 45/00 A 61 K 37/00 |
| A | EP - A - 0 251 631 (ANDERSON, MARK E.) | 1-10 | |

The present search report has been drawn up for all claims

| Place of search | Date of completion of the search | Examiner |
|---|---|---|
| VIENNA | 20-05-1992 | SCHARF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | * Page 3, column 2, lines 53-57, page 4; column 1, lines 1-11 * | | |
| A | CHEMICAL ABSTRACTS, vol. 102, no. 21, May 27, 1985, Columbus, Ohio, USA GIULIAN, DANA et al. "Interleukin-1 stimulation of astroglial proliferation after brain injury." page 457, column 1, abstract-no. 183 584k & Science 1985, 228(4698), 407-9 | 1-10 | |
| A | CHEMICAL ABSTRACTS, vol. 104, no. 15, April 14, 1986, Columbus, Ohio, USA GIULIAN, DANA et al. "Interleukin-1 as a mediator of brain cell growth." page 518, column 2, abstract-no. 127 759a & Prog.Leukocyte Biol. 1985, 2, 133-42 | 1-10 | TECHNICAL FIELDS SEARCHED (Int. Cl.5) |

The present search report has been drawn up for all claims

| Place of search | Date of completion of the search | Examiner |
|---|---|---|
| VIENNA | 20-05-1992 | SCHARF |